# EUROPEAN PATENT APPLICATION

(11) **EP 4 711 381 A1**
(43) Date of publication of application: **18.03.2026**
(21) Application number: 24806591.4
(22) Date of filing: 15.05.2024
(51) Int. Cl.: C07K 14/47, C12N 15/12, C12Q 1/6886, C12N 15/85, A61K 38/16, A61P 35/00

(54) **NEW MICRO PEPTIDE HMBJ AND USE THEREOF**

(30) Priority: 15.05.2023 CN 202310539447
(71) Applicant: Nanjing Anji Biotechnology Co., Ltd., Nanjing, Jiangsu 210000 (CN)
(72) Inventor: XU, Hanmei, Nanjing, Jiangsu 210000 (CN); LI, Bangjie, Nanjing, Jiangsu 210000 (CN)
(74) Representative: karo IP
(86) International application number: PCT/CN2024/093256
(87) International publication number: WO 2024/235241

(57) **Abstract**

This application discloses new micropeptide HMBJ and use thereof, and relates to the field of biological medicine. The new micropeptide HMBJ is newly discovered human endogenous polypeptide and is coded by human lncRNA ENSG00000272654 and/or an ORF sequence contained therein. The inventor has for the first time discovered that the lncRNA molecule ENSG00000272654 is significantly associated with organ fibrosis, and specifically, expressed at a low level in cells and/or tissues of multiple organ fibroses. In addition, the synthetic peptide prepared based on the sequence of HMBJ exhibits significant inhibitory effect on key indicators of multiple organ fibroses, including collagen type I α1 chain gene, transforming growth factor β1, and connective tissue growth factor, indicating that the lncRNA ENSG00000272654, the ORF sequence, and the micropeptide HMBJ translated from the ORF sequence hold significant clinical diagnosis and treatment value for organ fibrosis.

## Description

### TECHNICAL FIELD

This application relates to the field of biological medicine, and specifically, to new micropeptide HMBJ, lncRNA and/or ORF for coding the new micropeptide HMBJ, and use thereof in diagnosis or auxiliary diagnosis and treatment or auxiliary treatment of multiple organ fibroses.

### BACKGROUND

Long non-coding RNA (lncRNA) is a functional RNA molecule composed of more than 200 nucleotides, which is not translatable into protein. Related studies have indicated that lncRNA plays a crucial role in epigenetic regulation, cell cycle control, and cell differentiation regulation, contributing to major human health disorders including cancer, neurodegenerative diseases, and the like. With the rapid advancement of molecular biology, computational biology, and deep sequencing technology, many previously unannotated non-canonical short open reading frames (ORFs) hidden within the long non-coding RNA have been identified as possessing a function of coding functional protein. Micropeptide is a translation product with a length of 2 to 100 codons, coded by short open reading frames (sORFs).

Organ fibrosis is a fibrotic disease that can occur in multiple organs, with primary pathological changes of increased fibrous connective tissue and reduced parenchymal cells in organ tissue. Persistent fibrosis in an organ can disrupt the structure and function of the organ, further leading to organ failure and malignant tumors. The persistent fibrosis is considered as a tumor-like lesion between benign and malignant conditions, posing a severe threat to human health and life.

Discovery and study of micropeptide associated with organ fibrosis are of significant importance for development of novel and effective disease diagnosis and treatment, and are of great reference and development value for exploring new application fields. However, no micropeptide related to the field of organ fibrosis research has been reported.

### SUMMARY

### 1. Invention purpose

A first objective of this application is to provide a lncRNA and a nucleotide sequence thereof, where the sequence is expressed at a low level in organ fibrosis cells and/or tissues.

A second objective of this application is to provide an ORF and a nucleotide sequence thereof, where the sequence of the ORF is in the foregoing lncRNA and can code new micropeptide HMBJ.

A third objective of this application is to provide new micropeptide HMBJ and an amino acid sequence thereof, where the new micropeptide HMBJ is newly discovered human endogenous polypeptide.

A fourth objective of this application is to provide use of the lncRNA in preparation of a reagent for diagnosis or auxiliary diagnosis of organ fibrosis and/or in preparation of a drug for treatment or auxiliary treatment of organ fibrosis.

A fifth objective of this application is to provide use of the ORF sequence in the lncRNA in preparation of a reagent for diagnosis or auxiliary diagnosis of organ fibrosis and/or in preparation of a drug for treatment or auxiliary treatment of organ fibrosis.

A sixth objective of this application is to provide use of the new micropeptide HMBJ or the nucleotide for coding the new micropeptide HMBJ in preparation of a drug for treatment or auxiliary treatment of organ fibrosis.

### 2. Technical solution

To achieve the foregoing invention objectives, this application provides the following technical solutions:
This application provides a lncRNA molecule ENSG00000272654 with a nucleotide sequence as set forth in SEQ ID NO. 1. Through analysis of high-throughput sequencing data for multiple organ fibroses, the applicant has for the first time discovered that the lncRNA molecule ENSG00000272654 is significantly associated with organ fibrosis, and specifically, expressed at a low level in cells and/or tissues of multiple organ fibroses. To date, no related research has been reported.

This application further provides an open reading frame (ORF) of the lncRNA molecule ENSG00000272654, with a nucleotide sequence as set forth in SEQ ID NO. 2. Through analysis of translation features of the open reading frame of the lncRNA molecule ENSG00000272654, the applicant has discovered that the open reading frame has potential to code functional protein. In addition, through endogenous coding validation by gene editing, immunoprecipitation, LC-MS/MS, and another method, it is verified that the lncRNA molecule ENSG00000272654 has a coding sequence (ORF) and the sequence has not been reported.

This application further provides new micropeptide HMBJ with an amino acid sequence containing a sequence as set forth in SEQ ID NO. 3. The new micropeptide HMBJ is coded by human lncRNA ENSG00000272654 and is newly discovered human endogenous polypeptide.

This application further provides a nucleotide. The nucleotide codes the foregoing new micropeptide HMBJ. A sequence of the nucleotide is not limited to the lncRNA molecule ENSG00000272654 or the ORF contained therein. A person skilled in the art can design a corresponding nucleotide sequence based on the amino acid sequence of the new micropeptide HMBJ.

This application further provides a recombinant vector. The recombinant vector includes the sequence of the nucleotide for coding the new micropeptide HMBJ. The recombinant vector may be a vector known by a person skilled in the art, such as a plasmid or a viral vector.

This application further provides use of the new micropeptide HMBJ, the nucleotide for coding the new micropeptide HMBJ, or the recombinant vector in preparation of a drug for treatment or auxiliary treatment of organ fibrosis. The applicant has verified that the new micropeptide HMBJ exhibits significant inhibitory effect on an organ fibrosis cell model, and therefore can be used for treatment or auxiliary treatment of organ fibrosis.

Further, the organ fibrosis includes liver fibrosis, renal fibrosis, pulmonary fibrosis, and/or the like.

This application further provides use of the lncRNA molecule ENSG00000272654 and/or the ORF contained therein in preparation of a reagent for diagnosis or auxiliary diagnosis of organ fibrosis.

Further, the reagent for diagnosis or auxiliary diagnosis of organ fibrosis includes a primer for specifically amplifying the lncRNA molecule ENSG00000272654 or the ORF contained therein.

Further, the specific primer pair contains the following nucleotide sequences:
F: CAAGGGATGGAGGCGAAACT; and
R: CCACAGTGCTGGTCAAGTCA.

Further, the organ fibrosis includes liver fibrosis, renal fibrosis, and/or pulmonary fibrosis.

This application further provides a pharmaceutical composition for treatment or auxiliary treatment of organ fibrosis. The pharmaceutical composition contains at least the new micropeptide HMBJ, the nucleotide for coding the new micropeptide HMBJ, or the recombinant vector, and a pharmaceutically acceptable carrier.

This application further provides a kit for diagnosis or auxiliary diagnosis of organ fibrosis. The kit includes a primer for specifically amplifying the lncRNA molecule ENSG00000272654 or the ORF contained therein.

Further, the specific primer pair contains the following nucleotide sequences:
F: CAAGGGATGGAGGCGAAACT; and
R: CCACAGTGCTGGTCAAGTCA.

### 3. Beneficial effects

Compared with the related art, this application has the following beneficial effects:
(1) This application provides a lncRNA and a nucleotide sequence thereof. Through analysis of sequencing data for multiple organ fibroses, it is discovered that the lncRNA molecule ENSG00000272654 is expressed at a low level in the organ fibroses. Literature searches revealed that no related research was reported. The lncRNA molecule can be used for diagnosis or auxiliary diagnosis and treatment or auxiliary treatment of organ fibrosis, or can be used in preparation of a reagent for diagnosis or auxiliary diagnosis and treatment or auxiliary treatment of organ fibrosis.
(2) This application provides a nucleotide sequence of an open reading frame of the lncRNA. The nucleotide sequence can code new micropeptide HMBJ. Database (BLAST and UniProt) and literature searches revealed no protein or polypeptide fragments having a sequence homologous with that of the micropeptide HMBJ. The nucleotide sequence can be used for coding the new micropeptide HMBJ, or can be used for diagnosis or auxiliary diagnosis and treatment or auxiliary treatment of organ fibrosis, or can be used in preparation of a reagent for diagnosis or auxiliary diagnosis and treatment or auxiliary treatment of organ fibrosis.
(3) This application provides new micropeptide HMBJ with a new amino acid sequence. Database (BLAST and UniProt) and literature searches revealed no protein or polypeptide fragments having a sequence homologous with that of the micropeptide HMBJ. The new micropeptide HMBJ is composed of 71 amino acids. In vitro, the synthetic micropeptide HMBJ exhibits significant inhibitory effect on key indicators of human liver fibrosis, renal fibrosis, and pulmonary fibrosis, including collagen type I α1 chain gene (Col1a1), transforming growth factor β1 (TGFβ1), and connective tissue growth factor (CTGF). The new micropeptide HMBJ can also be used as a drug for treatment of another organ fibrosis, greatly broadening a therapeutic spectrum of the micropeptide, and offering new ideas for development of drugs for organ fibrosis.
(4) According to this application, the new synthetic micropeptide HMBJ is obtained by using a solid-phase synthesis method based on the amino acid sequence of the new micropeptide HMBJ. The method mainly includes polypeptide synthesis, polypeptide purification, polypeptide purity detection, and mass spectrum identification. Three organ fibrosis cell models are established. For example, 10 nmol recombinant human TGFβ1 (Peprotech) stimulating factor and different concentrations of the synthetic micropeptide HMBJ are separately added to human hepatic stellate cells LX-2, human non-small cell lung cancer cells A549, and human renal proximal tubule cells HK2, followed by co-incubation for 24 h. Then, total RNA is extracted from collected cell samples by using a TRIzol reagent. Then, the extracted total RNA is reverse-transcribed by using a reverse transcription reagent, and then mRNA expressions of gene Col1a1, TGFβ1, and CTGF in the three organ fibrosis cell models are detected by real-time fluorescence quantification PCR. The results show that the synthetic micropeptide HMBJ exhibits significant inhibitory effect on the three organ fibrosis cell models.

### BRIEF DESCRIPTION OF THE DRAWINGS

FIG. 1 is a volcano plot showing low expression of ENSG00000272654 in organ fibrosis by differential analysis;
FIG. 2 shows Sanger sequencing of monoclonal cells with a Flag tag inserted into micropeptide HMBJ by gene editing;
FIG. 3 shows expression of a target band of micropeptide HMBJ detected by immunoprecipitation;
FIG. 4 is a protein spectrum of micropeptide HMBJ detected by LC-MS/MS;
FIG. 5 shows melting curves of four organ fibrosis gene primers detected for specificity by qPCR;
FIG. 6 shows gene marker detection carried out on a human liver fibrosis cell model by qPCR;
FIG. 7 shows gene marker detection carried out on a human renal fibrosis cell model by qPCR;
FIG. 8 shows gene marker detection carried out on a human pulmonary fibrosis cell model by qPCR;
FIG. 9 shows results of liquid chromatography of synthetic micropeptide HMBJ;
FIG. 10 shows results of mass spectrometry of synthetic micropeptide HMBJ;
FIG. 11 shows inhibitory effect of micropeptide HMBJ on a human liver fibrosis cell model detected by qPCR;
FIG. 12 shows inhibitory effect of micropeptide HMBJ on a human renal fibrosis cell model detected by qPCR; and
FIG. 13 shows inhibitory effect of micropeptide HMBJ on a human pulmonary fibrosis cell model detected by qPCR.

### DETAILED DESCRIPTION

The following further describes this application with reference to specific examples.

Unless otherwise defined, all technical and scientific terms used in this specification have the same meaning as would normally be understood by a person skilled in the art of this application. The term "and/or" used in this specification includes any and all combinations of one or more related listed items.

Steps in the examples without specific conditions indicated are performed under conventional conditions or conditions provided by manufacturers. Reagents or instruments used with no indication of manufacturers are conventional products that are commercially available.

As used herein, the term "about" is used to provide flexibility and imprecision associated with a given term, measure, or value. A person skilled in the art can readily determine a degree of flexibility of a specific variable.

As used herein, the term "at least one of ..." is intended to be the same as "one or more of ...". For example, "at least one of A, B, and C" expressly includes only A, only B, only C, and a combination thereof.

A concentration, an amount, and other numerical data may be presented herein in a range format. It should be understood that such range format is used for convenience and brevity only, and should be flexibly explained as including not only a value explicitly described as a range limit, but also all individual values or subranges included in the range, as if each value and subrange are explicitly described. For example, a range of values from about 1 to about 4.5 should be explained as including not only the explicitly described limit values of about 1 and about 4.5, but also individual numbers (such as 2, 3, and 4) and subranges (such as 1 to 3, and 2 to 4). The same principle is suitable for a range that describes only one value, for example, "less than about 4.5" should be explained as including all of the foregoing values and ranges. In addition, such explanation should be suitable for all described ranges or characteristics regardless of breadth.

In an example, methods for endogenous coding validation on the micropeptide HMBJ include gene editing, immunoprecipitation, and LC-MS/MS, specifically:
a Flag tag is inserted correctly at a location of a genome of the micropeptide HMBJ by gene editing;
the generation of a target band is observed by immunoprecipitation with chemiluminescence; and
a protein spectrum of the micropeptide HMBJ is detected by LC-MS/MS.

In an example, gene marker detection is carried out on organ fibrosis cell models by real-time fluorescence quantification PCR, specifically:
human hepatic stellate cells LX-2, human non-small cell lung cancer cells A549, and human renal proximal tubule cells HK2 are respectively spread in well plates, and different concentrations of recombinant human TGFβ1 (Peprotech) stimulating factors are added; and
after continuous stimulation for 24 h and 48 h separately, mRNA expressions of gene Col1a1 and α-SMA are detected separately by real-time fluorescence quantification PCR to determine whether the cell models are established successfully.

In an example, new micropeptide HMBJ is obtained by solid-phase synthesis, and the synthetic peptide is subjected to a quality test, specifically:
peptide resin synthesis includes decapping with hexahydropyridine/N,N-dimethylformamide (DMF), then washing resin with DMF, then adding a condensing agent for thorough reaction in a reactor, and finally washing the resin fully with DMF to remove a by-product;
a lysis solution is added to fully lyse polypeptide, and the polypeptide is separated from the resin by using a sand core funnel;
anhydrous ether is added to precipitate the polypeptide, centrifugation is carried out, a supernatant is removed, and then the polypeptide is washed with anhydrous ether and then dried to obtain crude polypeptide;
the crude polypeptide is dissolved in deionized water, filtered, and purified by semi-preparative high-performance liquid chromatography; and
purity detection is carried out on the polypeptide by analytical RP-HPLC.

In an example, a method for detecting inhibitory effect of the micropeptide HMBJ on fibrosis cell models by real-time fluorescence quantification PCR includes the following steps:
human hepatic stellate cells LX-2, human non-small cell lung cancer cells A549, or human renal proximal tubule cells HK2 are counted and spread in a well plate;
except for a blank group, 10 nmol recombinant human TGFβ1 (Peprotech) stimulating factor is added to carry out continuous stimulation for 24 h;
total RNA is extracted from collected cell samples by using a TRIzol reagent, the extracted total RNA is reverse-transcribed by using a reverse transcription reagent, and then a qPCR reaction is carried out by using a qPCR pre-mixed solution; and
mRNA expressions of gene Col1a1, TGFβ1, and CTGF are detected separately by using a real-time fluorescence quantification PCR instrument.

### Example 1

In this example, expression levels of the gene ENSG00000272654 in multiple organ fibroses were analyzed.

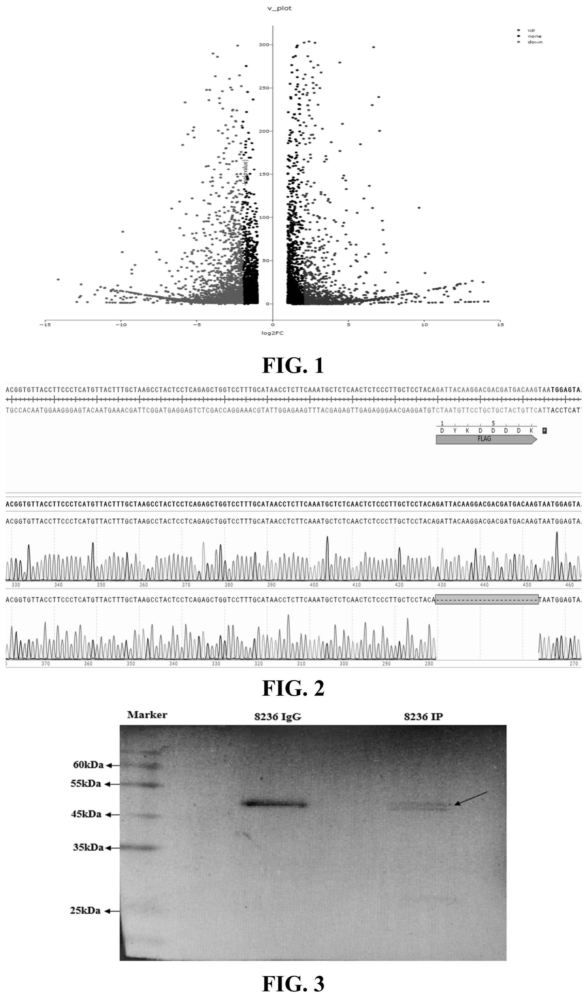

Fibrosis and *Homo sapiens* were used as keywords to search in the NCBI and ENA databases. High-throughput sequencing data associated with multiple organ fibroses was batch-downloaded by using the Aspera command, including human liver fibrosis and normal cell data (accession number: PRJNA638727), human liver fibrosis patient and healthy tissue data (accession number: PRJEB27201), human renal fibrosis and normal cell data (accession number: GSE23338), and human pulmonary fibrosis patient and healthy tissue data (accession number: PRJNA878759). The downloaded sequencing data was then processed and analyzed. First, the original data was filtered by using the Fastp software package (v0.18.0) to remove reads containing adapter sequences and low-quality bases. Then, the data was aligned with human tRNA and rRNA sequences by using the Bowtie software package (v1.3.0), and unsuccessfully aligned data was aligned with the human reference genome (GRCh38.Ensembl98) by using the HISAT2 software package (v2.2.1). Finally, reads with an alignment success rate greater than 90% were used as data for subsequent analysis, then genes and transcripts were assembled and quantified by using the StringTie software package (v1.2.2), and then differential gene expression analysis was carried out by using the DESeq2 software package (v1.38.3). The results show that the gene ENSG00000272654 exhibits significance padj < 0.05 and a fold change (log2FoldChange) < -1, indicating that the gene is expressed at a low level in multiple organ fibroses. The expression differences are shown in FIG. 1.

### Example 2

In this example, endogenous detection was carried out for a coding capability of the micropeptide HMBJ, including gene editing, immunoprecipitation, and LC-MS/MS.

A gene sequence of ENSG00000272654 was downloaded from the Ensembl database. Corresponding sgRNA plasmids and donor fragments containing a Flag tag were designed. The sgRNA plasmids and donor fragments were transfected into 293T cells by using a lipo3000 liposome transfection reagent, and then the cells were observed for fluorescence after 48 h of transfection. The cells were collected through trypsin digestion, centrifuged to remove a supernatant, then resuspended in a basal medium, and then sorted by flow cytometry. The sorted cells were counted, followed by monoclonal plating and Sanger sequencing to identify monoclonal cells with the Flag tag correctly inserted, as shown in FIG. 2.

The cells were rinsed with PBS twice, collected through trypsin digestion, and centrifuged to remove a supernatant. Then, a cell precipitate was resuspended and lysed in a RIPA lysis solution on ice for 30 min, and then centrifuged at 12000 g for 15 min. A supernatant was collected. A Flag antibody and agarose gel were added to form a protein-antibody-gel complex. The complex was centrifuged at 6000 g for 1 min, and the gel was removed. A 1×SDS loading buffer was added to obtain a mixture. The mixture was mixed uniformly and boiled to denature for 10 min. Total protein was separated by using 12% SDS-PAGE gel and transferred to a PVDF membrane. Then, 5% nonfat dry milk was added for blocking at room temperature for 3 h, followed by washing with TBST for five times, 10 min each time. A Flag primary antibody was added for incubation at 4°C overnight, followed by washing with TBST for five times, 10 min each time. A secondary antibody was added for incubation at room temperature for 1 h, followed by washing with TBST for five times, 10 min each time. An ECL ultra-sensitive chemiluminescent reagent was added for development, and a Tannon imaging system was used to detect whether a target band was generated. The results are shown in FIG. 3.

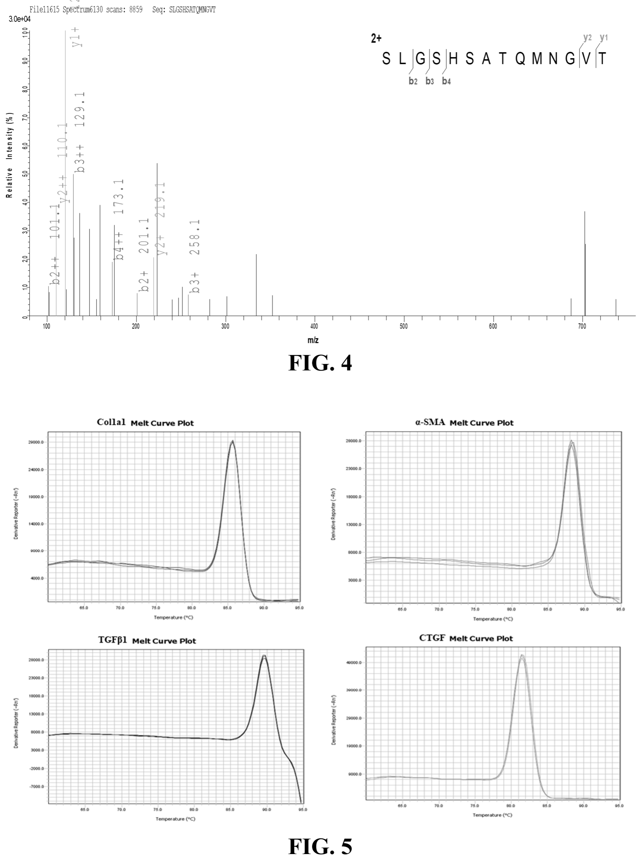

To further validate the coding capability of the micropeptide HMBJ, the SDS-PAGE gel containing target protein in the foregoing step was stained with Coomassie brilliant blue, and the visible target band was separated from the gel and subjected to LC-MS/MS (liquid chromatography-tandem quadrupole mass spectrometer). to obtain mass spectrometry results of the target micropeptide, as shown in FIG. 4.

It is found through gene editing, immunoprecipitation, and mass spectrometry that, after the Flag tag is inserted into the genomic sequence of the target micropeptide, the target band of the micropeptide HMBJ is observed, and the peptide fragment of the micropeptide HMBJ appears on the mass spectrum, fully indicating that the new micropeptide HMBJ has an endogenous coding capability.

### Example 3

In this example, gene marker detection was carried out on three organ fibrosis cell models by real-time fluorescence quantification PCR.

### (1) Primer design

Key indicators of the organ fibrosis cell models include collagen type I α1 chain gene (Col1a1), transforming growth factor β1 (TGFβ1), and connective tissue growth factor (CTGF). Corresponding primers were designed by Primer Premier 5.0, including Col1a1 upstream and downstream primers, TGFβ1 upstream and downstream primers, CTGF upstream and downstream primers, and α-SMA upstream and downstream primers. Total RNA was extracted from collected cell samples according to the instruction of the TRIzol reagent from Life Invitrogen. Then, the purity and concentration of the extracted RNA were quantified by using a NanoDrop 2000 micro-volume spectrophotometer, and a quality test was carried out with agarose gel to ensure RNA integrity. The extracted total RNA was reverse-transcribed by using a reverse transcription reagent HiScript^{®} III RT SuperMix for qPCR (+gDNA wiper), and then a qPCR reaction was carried out by using a qPCR pre-mixed solution ChamQ SYBR qPCR Master Mix. A reaction system is shown in the following table:

**Table 1: qPCR reaction system**

| Reagent | Volume (µL) |
|---|---|
| 2 × ChamQ SYBR qPCR Master Mix | 10.0 |
| Primer F | 0.4 |
| Primer R | 0.4 |
| cDNA | 1.0 |
| ddH₂O | 8.2 |
| Total | 20 |

The foregoing components were mixed uniformly and underwent the reaction under the following conditions: pre-denaturation at 95°C for 30s, 95°C for 10s, and 60°C for 30s, for a total of 50 cycles. After the qPCR reaction ended, a Cq value of each sample well was recorded. A maximum Cq value variation among triplicate sample wells was ensured to be ≤ 0.5 to ensure data accuracy. Whether a melting curve of the designed primer had a single peak was observed to determine reaction specificity, as shown in FIG. 5.

### (2) Model establishment

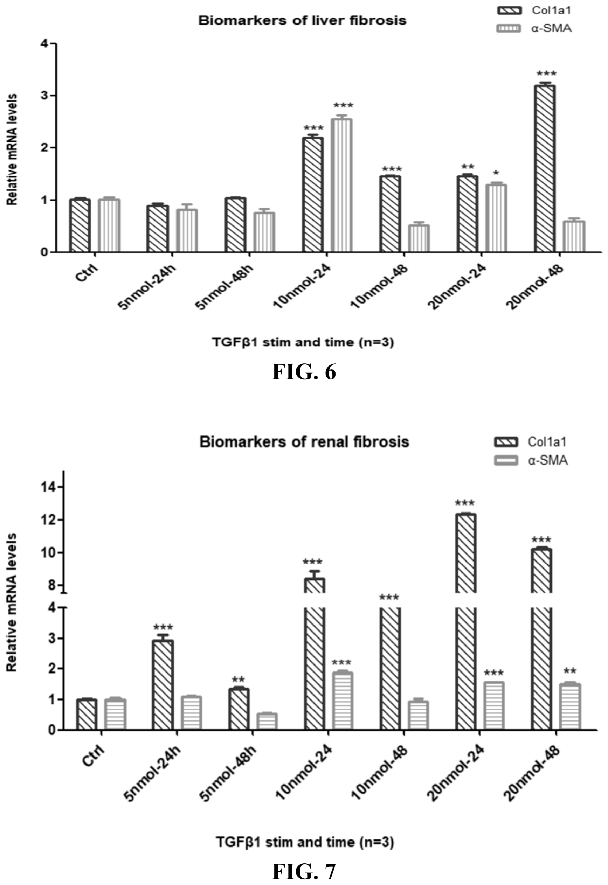

Human hepatic stellate cells LX-2, human non-small cell lung cancer cells A549, and human renal proximal tubule cells HK2 were separately cultured in a cell incubator at 37°C under 5% CO₂ until a cell density was 80% or more. The cells were collected through trypsin digestion and centrifuged to remove a supernatant. Then, the cells were resuspended in a complete medium and counted by using a cell counter. The cells were adjusted to a concentration of 1.5×10⁵ cells/mL, then inoculated into a six-well cell culture plate, and placed in a cell incubator at 37°C under 5% CO₂ for culture. After 24 h of cell culture, the medium was removed, different concentrations of recombinant human TGFβ1 (Peprotech) stimulating factors were added to carry out continuous stimulation for 24 h and 48 h, and then mRNA expressions of gene Collal and α-SMA were detected separately by using a qPCR method, as shown in FIG. 6, FIG. 7, and FIG. 8.

### Example 4

In this example, micropeptide HMBJ was obtained by solid-phase synthesis, and the synthetic micropeptide was subjected to a quality test.

In this example, the micropeptide HMBJ was synthesized by polypeptide solid-phase synthesis, the synthesized micropeptide HMBJ was separated and purified by preparative HPLC, and purity of the micropeptide HMBJ was measured by analytical RP-HPLC. Through the polypeptide solid-phase synthesis, Fmoc-Thr-wang-resin, as a starting material, was sequentially coupled to a target sequence with protected amino acids until the last amino acid, followed by thorough washing, lysis, and post-treatment, to obtain crude polypeptide. The crude polypeptide was dissolved, purified by preparative HPLC, concentrated, and freeze-dried, to obtain pure polypeptide. Specific reaction steps are as follows:

### (1) Peptide resin synthesis

1 mmol Fmoc-Thr-wang-resin was weighed and transferred into a glass sand-core reaction column. 20 mL of CH₂Cl₂ was added to fully expand the resin.
a. Decapping: 20 mL of 20% hexahydropyridine/N,N-dimethylformamide (DMF) decapping solution was added to react for a period of time. Then, the decapping solution was removed, followed by washing with DMF once. Then, 20 mL of decapping solution was added again to react to remove the Fmoc protecting group.
b. Washing: The decapping solution was removed, and the resin was washed with DMF to fully remove a by-product.
c. Condensation: Protected amino acids for coupling and an activator were dissolved in DMF and a condensing agent, for thorough reaction in a reactor.
d. Washing: The reaction solution was removed, and the resin was washed fully with DMF to remove a by-product.

### (2) Lysis

The dried resin was transferred in a conical flask, a lysis solution was added to fully lyse the synthesized polypeptide, and the polypeptide was separated from the resin by using a sand core funnel. The lysis solution contains the following components in a volume ratio: trifluoroacetic acid:phenol:water:thioanisole:EDT = 90:3:3:2:2.

### (3) Post-treatment

The lysis solution was added to anhydrous ether in an ice bath and stirred slowly to precipitate the polypeptide. Centrifugation was carried out. A supernatant was removed. Then, the polypeptide was washed with anhydrous ether and then dried to obtain crude polypeptide.

### (4) Purification

a. Dissolution: The crude polypeptide was dissolved in deionized water and filtered by using a filter membrane with a pore size of 0.45 µm.
b. Preparation: Purification was carried out by semi-preparative HPLC with a mobile phase A: 0.1% TFA (acetonitrile) and a mobile phase B: 0.1% TFA (water). A purification system is shown in the following table:

**Table 2: Polypeptide purification system**

| Time (min) | Flow rate (mL/min) | A% | B% | Wavelength (nm) |
|---|---|---|---|---|
| 0 | 10 | 15 | 85 | 220 |
| 50 | 10 | 50 | 50 | 220 |

A solution at an ultraviolet wavelength of 220 nm was collected. It is determined through MS that a molecular weight of the solution is consistent with a theoretical value. Through HPLC analysis, the purity of the solution is greater than 95%. The qualified solution was concentrated under reduced pressure at 37°C by using a rotary evaporator to remove the organic solvent, then transferred into a freeze-drying tray and frozen at -80°C, and then freeze-dried by using a freeze-dryer, to obtain pure polypeptide.

### (5) Purity detection

The freeze-dried purified product was collected and subjected to polypeptide purity detection by analytical RP-HPLC under the following analysis conditions: mobile phases: 0.1% TFA (acetonitrile) and 0.1% TFA (water); and loading volume: 10 µL. A specific reaction system is shown in the following table:

**Table 3: Purity detection system**

| Time (min) | Flow rate (mL/min) | A% | B% | Wavelength (nm) |
|---|---|---|---|---|
| 0.0 | 1 | 15 | 85 | 220 |
| 25.0 | 1 | 70 | 30 | 220 |
| 25.1 | 1 | 100 | 0 | 220 |
| 30.0 | 1 | Stop | | 220 |

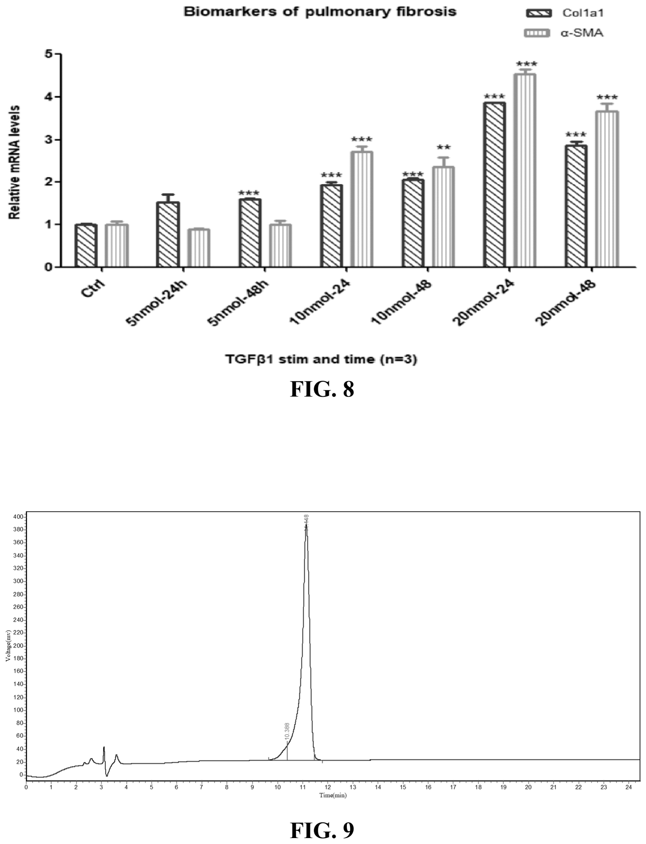

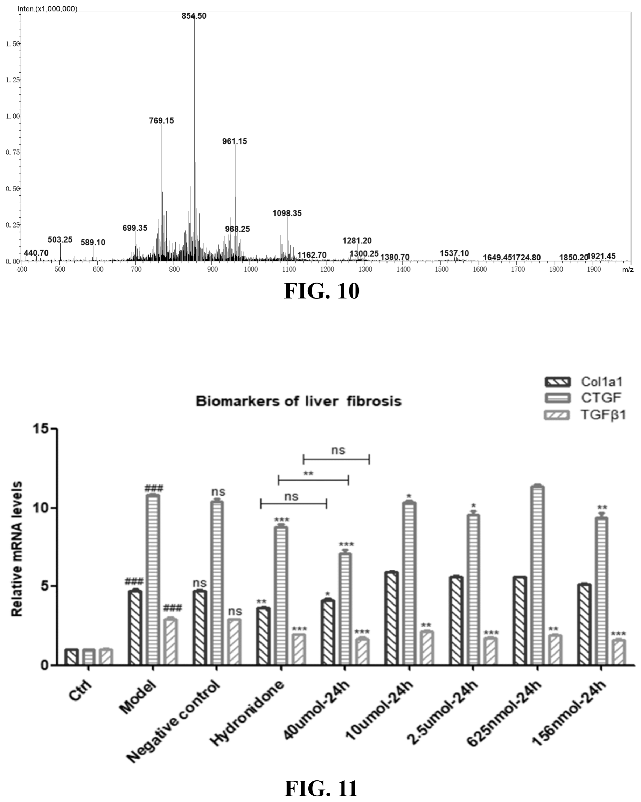

The synthesized polypeptide was subjected to purity identification by reverse-phase liquid chromatography. The results show that the purity of the prepared micropeptide HMBJ reaches 95.86%, which is greater than 95%, as shown in FIG. 9 and FIG. 10, meeting requirements for subsequent experiments. Specific information is shown in the following table:

**Table 4: Purity detection results**

| Name | Peak area | Purity (%) |
|---|---|---|
| Micropeptide HMBJ | 8331005 | 95.86 |

### Example 5

In this example, inhibitory effect of the micropeptide HMBJ on a human liver fibrosis cell model was detected. Human hepatic stellate cells LX-2 were cultured in a cell incubator at 37°C under 5% CO₂ until a cell density was 80% or more. The cells were collected through trypsin digestion and centrifuged to remove a supernatant. Then, the cells were resuspended in a complete medium and counted by using a cell counter. The cells were adjusted to a concentration of 1.5×10⁵ cells/mL, then inoculated into a six-well cell culture plate, and placed in a cell incubator at 37°C under 5% CO₂ for culture. After 24 h of cell culture, the medium was removed. 2‰ DMSO solvent was added to obtain a negative control, and 20 µmol hydronidone was added to obtain a positive control. In other groups, 40 µmol, 10 µmol, 2.5 µmol, 625 nmol, and 156 nmol synthetic peptides HMBJ were added in equal proportion to obtain administration groups. After 30 minutes, except for a blank control, 10 nmol recombinant human TGFβ1 (Peprotech) stimulating factor was added in each group to carry out continuous stimulation for 24 h. Then, mRNA expressions of gene Col1a1, TGFβ1, and CTGF were detected separately, as shown in FIG. 11.

### Example 6

In this example, inhibitory effect of the micropeptide HMBJ on a human renal fibrosis cell model was detected.

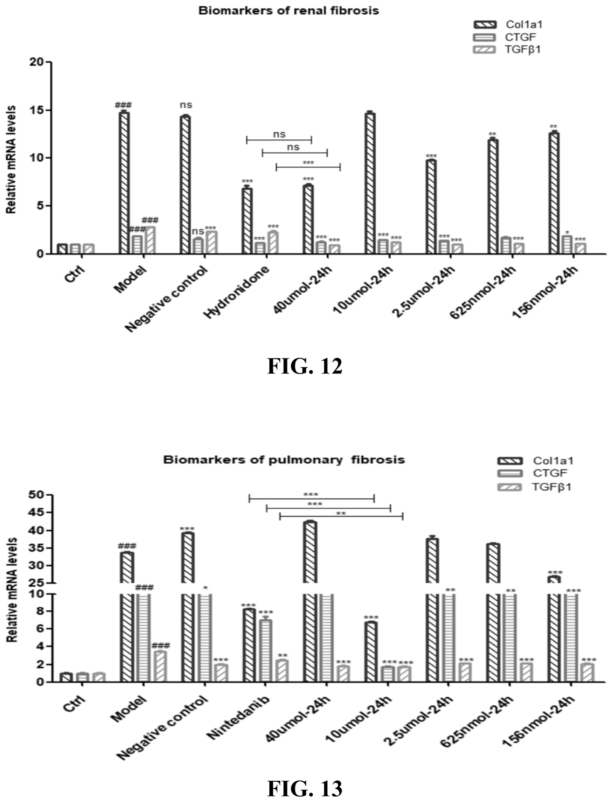

Human renal proximal tubule cells HK2 were cultured in a cell incubator at 37°C under 5% CO₂ until a cell density was 80% or more. The cells were collected through trypsin digestion and centrifuged to remove a supernatant. Then, the cells were resuspended in a complete medium and counted by using a cell counter. The cells were adjusted to a concentration of 1.5×10⁵ cells/mL, then inoculated into a six-well cell culture plate, and placed in a cell incubator at 37°C under 5% CO₂ for culture. After 24 h of cell culture, the medium was removed. 2‰ DMSO solvent was added to obtain a negative control, and 20 µmol hydronidone was added to obtain a positive control. In other groups, 40 µmol, 10 µmol, 2.5 µmol, 625 nmol, and 156 nmol synthetic peptides HMBJ were added in equal proportion to obtain administration groups. After 30 minutes, except for a blank control, 10 nmol recombinant human TGFβ1 (Peprotech) stimulating factor was added in each group to carry out continuous stimulation for 24 h. Then, mRNA expressions of gene Col1a1, TGFβ1, and CTGF were detected separately, as shown in FIG. 12.

### Example 7

In this example, inhibitory effect of the micropeptide HMBJ on a human pulmonary fibrosis cell model was detected.

Human non-small cell lung cancer cells A549 were cultured in a cell incubator at 37°C under 5% CO₂ until a cell density was 80% or more. The cells were collected through trypsin digestion and centrifuged to remove a supernatant. Then, the cells were resuspended in a complete medium and counted by using a cell counter. The cells were adjusted to a concentration of 1.5×10⁵ cells/mL, then inoculated into a six-well cell culture plate, and placed in a cell incubator at 37°C under 5% CO₂ for culture. After 24 h of cell culture, the medium was removed. 2‰ DMSO solvent was added to obtain a negative control, and 2 µmol Nintedanib was added to obtain a positive control. In other groups, 40 µmol, 10 µmol, 2.5 µmol, 625 nmol, and 156 nmol synthetic peptides HMBJ were added in equal proportion to obtain administration groups. After 30 minutes, except for a blank control, 10 nmol recombinant human TGFβ1 (Peprotech) stimulating factor was added in each group to carry out continuous stimulation for 24 h. Then, mRNA expressions of gene Col1a1, TGFβ1, and CTGF were detected separately, as shown in FIG. 13.

## Claims

1. New micropeptide HMBJ, wherein the new micropeptide HMBJ contains an amino acid sequence as set forth in SEQ ID NO. 3.

2. A nucleotide, wherein the nucleotide codes the new micropeptide HMBJ according to claim 1.

3. The nucleotide according to claim 2, wherein a sequence of the nucleotide comprises a lncRNA molecule ENSG00000272654 and/or an ORF contained therein, a nucleotide sequence of the lncRNA molecule ENSG00000272654 is as set forth in SEQ ID NO. 1, and a nucleotide sequence of the ORF contained in the lncRNA molecule ENSG00000272654 is as set forth in SEQ ID NO. 2.

4. Use of the nucleotide according to claim 3 in preparation of a reagent for diagnosis or auxiliary diagnosis of organ fibrosis, wherein the organ fibrosis comprises liver fibrosis, renal fibrosis, and/or pulmonary fibrosis.

5. The use of the nucleotide in preparation of the reagent for diagnosis or auxiliary diagnosis of the organ fibrosis according to claim 4, wherein the reagent for diagnosis or auxiliary diagnosis of the organ fibrosis comprises a primer for specifically amplifying the lncRNA molecule ENSG00000272654 or the ORF contained therein.

6. A recombinant vector, wherein the recombinant vector contains the nucleotide according to claim 2 or 3.

7. Use of the new micropeptide HMBJ according to claim 1, the nucleotide according to claim 2 or 3, or the recombinant vector according to claim 6 in preparation of a drug for treatment or auxiliary treatment of organ fibrosis, wherein the organ fibrosis comprises liver fibrosis, renal fibrosis, and/or pulmonary fibrosis.

8. A pharmaceutical composition for treatment or auxiliary treatment of organ fibrosis, wherein the pharmaceutical composition contains at least the new micropeptide HMBJ according to claim 1, the nucleotide according to claim 2 or 3, or the recombinant vector according to claim 6, and a pharmaceutically acceptable carrier.

9. A kit for diagnosis or auxiliary diagnosis of organ fibrosis, wherein the kit contains a specific primer pair for the sequence of the nucleotide according to claim 3.

10. The kit for diagnosis or auxiliary diagnosis of the organ fibrosis according to claim 9, wherein the specific primer pair contains the following nucleotide sequences:
F: CAAGGGATGGAGGCGAAACT; and
R: CCACAGTGCTGGTCAAGTCA.
